# EUROPÄISCHE PATENTSCHRIFT

(11) **EP 4 188 846 B1**
(45) Veröffentlichungstag und Bekanntmachung des Hinweises auf die Patenterteilung: **03.07.2024**
(21) Anmeldenummer: 21754703.3
(22) Anmeldetag: 19.07.2021
(51) Int. Cl.: B65G 47/14, B65G 59/06, B65G 65/00, B65B 3/00, A61M 5/32, A61M 5/34

(54) **BEREITSTELLUNGSVORRICHTUNG FÜR KANÜLEN, VERFAHREN ZUR BEREITSTELLUNG VON KANÜLEN, COMPUTERPROGRAMM UND COMPUTERLESBARES MEDIUM**
SUPPLY DEVICE FOR CANNULAS, METHOD FOR SUPPLYING CANNULAS, COMPUTER PROGRAM, AND COMPUTER-READABLE MEDIUM
DISPOSITIF DE FOURNITURE DE CANULES, PROCÉDÉ DE FOURNITURE DE CANULES, PROGRAMME INFORMATIQUE ET SUPPORT LISIBLE PAR ORDINATEUR

(30) Priorität: 27.07.2020 DE 102020119681
(43) Veröffentlichungstag der Anmeldung: 07.06.2023
(73) Patentinhaber: Zahoransky Automation & Molds GmbH, 79108 Freiburg (DE)
(72) Erfinder: BAUMANN, Frank, 77966 Kappel-Grafenhausen (DE); STEFAN, Simon, 79874 Breitnau (DE)
(74) Vertreter: Mertzlufft-Paufler, Cornelius
(86) Internationale Anmeldenummer: PCT/EP2021/070103
(87) Internationale Veröffentlichungsnummer: WO 2022/023097

(56) Entgegenhaltungen:
- EP-A1- 0 030 353
- DE-U1-202008 018 272
- US-A1- 2006 188 358

## Beschreibung

Die Erfindung betrifft Bereitstellungsvorrichtungen zur Bereitstellung von Kanülen, ein Verfahren zur Bereitstellung von Kanülen, ein Computerprogramm sowie ein computerlesbares Medium.

Beispielsweise bei der automatisierten Herstellung von Spritzen kann es erforderlich sein, Kanülen aus einem Vorrat zu vereinzeln und für nachgelagerte Prozessschritte bereitzustellen.

Aufgabe der Erfindung ist es, die Bereitstellung von Kanülen zu vereinfachen.

Zur Lösung der Aufgabe wird zunächst eine Bereitstellungsvorrichtung zur Bereitstellung von Kanülen vorgeschlagen, die die Merkmale des unabhängigen, auf eine derartige Bereitstellungsvorrichtung gerichteten Anspruchs aufweist. Zur Lösung der Aufgabe wird somit insbesondere eine Bereitstellungsvorrichtung zur Bereitstellung von Kanülen mit einem Kanülenmagazin und mit einem Entnahmeschlitten zur Entnahme von Kanülen aus dem Kanülenmagazin und zum Transport der Kanülen in eine Bereitstellungsposition vorgeschlagen. Erfindungsgemäß weist der Entnahmeschlitten zumindest eine Kanülenaufnahme für eine Kanüle auf, die zur Fixierung einer Kanüle in der Kanülenaufnahme mit einer Unterdruckquelle verbindbar oder verbunden ist.

Zur Entnahme von Kanülen aus dem Kanülenmagazin kann der Entnahmeschlitten in einer Entnahmebewegung, beispielsweise unterhalb des Kanülenmagazins, an einer Entnahmeöffnung des Kanülenmagazins vorbeigeführt werden. Unterstützt durch die Schwerkraft und/oder einen Drücker können die in dem Kanülenmagazin vorgehaltenen Kanülen dabei in die zumindest eine Kanülenaufnahme des Entnahmeschlittens gelangen. Mit Hilfe der Unterdruckquelle kann ein Unterdruck erzeugt werden, der über eine Verbindung zwischen der zumindest einen Kanülenaufnahme und der Unterdruckquelle auf die Kanülenaufnahme und eine darin platzierte Kanüle wirken kann.

Mit Hilfe des Unterdrucks, der auf die in der Kanülenaufnahme positionierte Kanüle wirkt, lässt sich die Kanüle zuverlässig und gleichsam schonend in der Kanülenaufnahme und damit an dem Entnahmeschlitten fixieren. So kann ein Verlust der Kanüle bei einer Bewegung des Entnahmeschlittens in eine Übergabeposition vermieden werden. Dies kann die Handhabung der Bereitstellungsvorrichtung vereinfachen, die Zuverlässigkeit der Bereitstellungsvorrichtung erhöhen und die Bereitstellung von Kanülen vereinfachen.

Ferner kann die Bereitstellungsvorrichtung mit einer Lagekorrekturvorrichtung ausgestattet sein, die dazu eingerichtet ist, eine in der zumindest einen Kanülenaufnahme angeordnete Kanüle in eine Soll-Lage auszurichten.

Bei der zuvor bereits erläuterten Entnahme einer Kanüle aus dem Kanülenmagazin ist nicht vorhersehbar, in welcher Lage die Kanüle in die zumindest eine Kanülenaufnahme des Entnahmeschlittens gelangt.

Um die Kanülen in einer definierten Lage an eine nachgelagerte Bearbeitungsvorrichtung zur Durchführung eines nachgelagerten Bearbeitungsschritts übergeben zu können, weist die Bereitstellungsvorrichtung die zuvor erwähnte Lagekorrekturvorrichtung vorgesehen. Mit ihr ist es möglich, eine in der zumindest einen Kanülenaufnahme des Entnahmeschlittens angeordnete Kanüle ausgehend von ihrer Ist-Lage in eine Soll-Lage auszurichten. Dies vereinfacht zunächst die Bereitstellung und außerdem auch die weitere Handhabung und Verarbeitung der Kanülen in nachgelagerten Bearbeitungsschritten.

Die Lagekorrekturvorrichtung kann insbesondere dazu eingerichtet sein, eine in der zumindest einen Kanülenaufnahme des Entnahmeschlittens angeordnete Kanüle um einen definierten Winkelbetrag zu drehen - insbesondere um ihre Längsachse - und/oder die Kanüle um eine definierte Strecke axial zu verschieben, um die Kanüle in eine Soll-Lage auszurichten. Die Lagekorrekturvorrichtung kann somit dazu eingerichtet sein, unterschiedliche Lageabweichungen der Kanülen von einer definierten Soll-Lage in der zumindest einen Kanülenaufnahme des Entnahmeschlittens zu beheben.

Die Lagekorrekturvorrichtung kann zumindest ein Ausrichtmittel zur Veränderung der Lage einer in der zumindest einen Kanülenaufnahme angeordneten Kanüle aufweisen. Als Ausrichtmittel kann die Lagekorrekturvorrichtung beispielsweise zumindest eine Klemmvorrichtung und/oder zumindest einen Antrieb umfassen. Mithilfe der zumindest einen Klemmvorrichtung kann eine Kanüle gegriffen und geklemmt werden. Mithilfe des Antriebs lässt sich die gegriffene und geklemmte Kanüle drehen und/oder axial verschieben, um sie in ihre Soll-Lage zu bewegen und eine etwaige Lageabweichung zu beheben.

Die Bereitstellungsvorrichtung kann wenigstens einen Sensor, insbesondere eine Kamera, zur Kontrolle der Anwesenheit und/oder zur Kontrolle der Lage einer in der zumindest einen Kanülenaufnahme des Entnahmeschlittens angeordneten Kanüle aufweisen. Mit Hilfe des zumindest einen Sensors lässt sich also feststellen, ob die zumindest eine Kanülenaufnahme des Entnahmeschlittens bei der Entnahme von Kanülen aus dem Kanülenmagazin tatsächlich mit einer Kanüle belegt wurde. Ferner ist es möglich, mit Hilfe des wenigstens einen Sensors auch die Lage einer Kanüle in der Kanülenaufnahme zu überwachen und festzustellen. Vorzugsweise ist jeder Kanülenaufnahme des Entnahmeschlittens jeweils ein derartiger Sensor zugeordnet. Der wenigstens eine Sensor kann an dem Entnahmeschlitten oder an einem Ständer der Bereitstellungsvorrichtung angeordnet sein.

Ergibt sich durch die Kontrolle der Lage der Kanüle in der zumindest einen Kanülenaufnahme eine Abweichung von einer Soll-Lage, ist es möglich, die zuvor bereits erwähnte Lagekorrekturvorrichtung der Bereitstellungsvorrichtung entsprechend anzusteuern und die Lageabweichung der Kanüle zu korrigieren.

Die Bereitstellungsvorrichtung kann zu diesem Zweck eine Steuereinheit aufweisen. Die Steuereinheit kann dazu eingerichtet sein, die Lagekorrekturvorrichtung in Abhängigkeit eines von dem wenigstens einen Sensor übermittelten Sensorsignals anzusteuern, um eine in der wenigstens einen Kanülenaufnahme angeordnete Kanüle in ihre Soll-Lage auszurichten und eine möglicherweise festgestellte Lageabweichung zu beheben.

Das Kanülenmagazin kann als Wechselmagazin ausgebildet sein. Die Bereitstellungsvorrichtung kann eine Halterung aufweisen, an der das Kanülenmagazin wechselbar befestigbar ist. Bei einem Modellwechsel der mit der Bereitstellungsvorrichtung bereitzustellenden Kanülen ist es auf diese Weise möglich, das als Wechselmagazin ausgebildete Kanülenmagazin von der Halterung abzunehmen und gegen ein mit anderen Kanülen befülltes Kanülenmagazin auszutauschen.

Bei einer Ausführungsform dieser Bereitstellungsvorrichtung ist vorgesehen, dass das Kanülenmagazin über eine formschlüssige Schiebeverbindung, insbesondere über eine Schwalbenschwanzverbindung, mit der Halterung verbunden ist.

Bei sämtlichen der zuvor erwähnten Ausführungsformen von Bereitstellungsvorrichtungen kann der Entnahmeschlitten zur Entnahme von Kanülen aus dem Kanülenmagazin in eine Übergabeposition bewegbar sein, in der er die entnommenen Kanülen für nachgelagerte Bearbeitungsschritte bereitstellt.

Sämtliche der zuvor erwähnten Bereitstellungsvorrichtungen können mit zumindest einem Entnahmegreifer ausgestattet sein. Mit Hilfe des Entnahmegreifers ist es möglich, Kanülen von dem Entnahmeschlitten zu entnehmen und an einen nachgelagerten Prozessschritt zu übergeben. Die Entnahme der Kanülen von dem Entnahmeschlitten kann hierbei vorzugsweise bei in Übergabeposition befindlichem Entnahmeschlitten erfolgen.

Vorteilhaft kann es in diesem Zusammenhang sein, wenn der Entnahmegreifer als Unterdruckgreifer ausgebildet ist. Der als Unterdruckgreifer ausgebildete Entnahmegreifer kann zumindest eine Greifaufnahme aufweisen, die mit einer Unterdruckquelle verbunden ist. So ist es möglich, mit dem Entnahmegreifer aufgenommene Kanülen mittels Unterdrucks in der jeweiligen Greifaufnahme zu halten.

Um alle mit dem Entnahmeschlitten aus dem Kanülenmagazin entnommene Kanülen gleichzeitig zu greifen und an einen nachgelagerten Prozessschritt übergeben zu können, ist es vorteilhaft, wenn der Entnahmegreifer eine der Anzahl der Kanülenaufnahmen an dem Entnahmeschlitten entsprechende Anzahl von Greifaufnahmen aufweist. Die Greifaufnahme/die Greifaufnahmen des Entnahmegreifers können hierbei mit einer, vorzugsweise mit einer gemeinsamen, Unterdruckquelle verbunden sein. Auf diese Weise kann jede der an dem Entnahmegreifer ausgebildeten Greifaufnahmen mit Unterdruck beaufschlagt werden, sodass sich die Kanülen besonders zuverlässig und schonend mit dem Entnahmegreifer halten lassen.

Erfindungsgemäß ist vorgesehen, dass die Bereitstellungsvorrichtung ein Hubmittel zum Anheben und Absenken eines in dem Kanülenmagazin befindlichen Kanülenvorrats aufweist. Insbesondere nach einem Magazinwechsel kann ein in dem Kanülenmagazin enthaltener Vorrat an losen Kanülen mit Hilfe des Hubmittels für eine nachfolgende Entnahme zunächst angehoben werden. Anschließend kann der Entnahmeschlitten unterhalb einer Entnahmeöffnung des Borstenmagazins positioniert werden. Sobald der Entnahmeschlitten seine Ausgangsposition unterhalb der Entnahmeöffnung eingenommen hat, kann der Vorrat loser Kanülen, der innerhalb des Kanülenmagazins bevorratet ist, mit Hilfe des Hubmittels behutsam auf die der Entnahmeöffnung zugewandten Oberseite des Entnahmeschlittens abgesenkt werden. Durch das behutsame Anheben und Absenken der losen Kanülen wird ein Verkanten der losen Kanülen innerhalb des Borstenmagazins verhindert.

Die von dem Hubmittel beim Anheben und Absenken durchgeführte Bewegung kann durch eine Führung vorgegeben und/oder geführt sein. Mit der Führung kann so eine vergleichsweise einfache Kurvensteuerung des Hubmittels vorgegeben sein. Eine derartige Kurvensteuerung ist besonders robust und auch vergleichsweise einfach zu realisieren.

Um ausreichend Platz für das Hubmittel bei in Ausgangsposition an dem Kanülenmagazin, insbesondere unterhalb des Kanülenmagazins, positioniertem Entnahmeschlitten zu schaffen und eine Kollision des Hubmittels mit dem Entnahmeschlitten zu vermeiden, ist erfindungsgemäß vorgesehen, dass der Entnahmeschlitten eine Längsnut aufweist, in die das Hubmittel eintauchen kann. Die Längsnut ist vorzugsweise randseitig offen, sodass das Hubmittel, zum Beispiel entlang der zuvor bereits erwähnten Führung, stirnseitig in die Längsnut eingefahren und durch eine Hubbewegung von unten in Richtung Kanülenmagazin angehoben werden kann. Das Hubmittel kann so auch bei unterhalb des Kanülenmagazins in Ausgangsposition angeordnetem Entnahmeschlitten in die zuvor erwähnte Längsnut bewegt werden. Dabei kann das Hubmittel vor die Entnahmeöffnung des Kanülenmagazins gelangen und durch eine entsprechende Hubbewegung gegen den darin angeordneten Kanülenvorrat bewegt werden. Durch eine weitere Hubbewegung kann der Kanülenvorrat von der Oberfläche des Entnahmeschlittens abgehoben werden. Bei angehobenem Kanülenvorrat kann zumindest ein Sicherungsmittel, beispielsweise zumindest ein Sicherungsstift vor die Entnahmeöffnung geschoben werden, um zu verhindern, dass Kanülen aus dem Kanülenmagazin fallen.

Der Entnahmeschlitten kann ferner eine Aussparung aufweisen, in die ein, beispielsweise der bereits zuvor erwähnte, Entnahmegreifer bei der Entnahme von Kanülen eintauchen kann. Dabei kann die Aussparung für den Entnahmegreifer quer oder rechtwinklig zu der zumindest einen Kanülenaufnahme ausgerichtet sein. Ferner kann die zumindest eine Aussparung so angeordnet sein, dass sie die zumindest eine Kanülenaufnahme in zwei Aufnahmeabschnitte unterteilt. Jeder der beiden Aufnahmeabschnitte einer Kanülenaufnahme kann mit einer oder der Unterdruckquelle verbunden sein. Vorzugsweise sind sämtliche Aufnahmeabschnitte sämtlicher Kanülenaufnahmen des Entnahmeschlittens mit einer gemeinsamen Unterdruckquelle verbunden.

Bei einer Ausführungsform der Bereitstellungsvorrichtung ist vorgesehen, dass in oder an der zumindest einen Kanülenaufnahme jeweils zumindest eine mit der Unterdruckquelle verbundene Ansaugstelle vorhanden ist. Über die zumindest eine Ansaugstelle in oder an der Kanülenaufnahme kann der Unterdruck der Unterdruckquelle zur Fixierung einer Kanüle auf eine in der Kanülenaufnahme positionierte Kanüle übertragen werden.

Bei einer Ausführungsform der Bereitstellungsvorrichtung ist vorgesehen, dass in oder an jeder Kanülenaufnahme der Bereitstellungsvorrichtung jeweils zumindest eine mit der Unterdruckquelle verbundene Ansaugstelle vorhanden ist.

Insbesondere bei einer Kanülenaufnahme, die in zwei oder mehrere Aufnahmeabschnitte unterteilt sind, kann es zweckmäßig sein, wenn in jedem Aufnahmeabschnitt der Kanülenaufnahme jeweils zumindest eine mit der Unterdruckquelle verbundene Ansaugstelle vorhanden ist. Auf diese Weise lassen sich Kanülen, die in der Kanülenaufnahme positioniert werden, besonders zuverlässig mittels Unterdrucks in der Kanülenaufnahme des Entnahmeschlittens fixieren.

Das zuvor bereits erwähnte Hubmittel kann durch eine Entnahmeöffnung des Kanülenmagazins in das Kanülenmagazin einführbar sein, um einen Kanülenvorrat in dem Kanülenmagazin anzuheben und beim Absenken des Hubmittels auch wieder abzusenken.

Das Kanülenmagazin kann zumindest ein Sicherungsmittel, beispielsweise ein Sicherungsstift, aufweisen. Mit Hilfe des zumindest einen Sicherungsmittels kann eine, beispielsweise die bereits zuvor erwähnte, Entnahmeöffnung des Kanülenmagazins verschließbar sein. Die Bereitstellungsvorrichtung kann eine Vorrichtung zum Entfernen und/oder Anbringen des zumindest einen Sicherungsmittels aufweisen. Diese Vorrichtung kann als Steckvorrichtung fungieren, mit dem ein Sicherungsmittel, insbesondere ein Sicherungsstift aus seiner Schließstellung vor und/oder innerhalb der Entnahmeöffnung des Kanülenmagazins gezogen und bei Bedarf auch wieder in seine Sicherungsstellung gesteckt werden kann.

Bei einer Ausführungsform der Bereitstellungsvorrichtung, die im Pendelbetrieb genutzt werden kann und daher besonders produktiv ist, ist vorgesehen, dass der Entnahmeschlitten zwei Entnahmefelder mit jeweils zumindest einer Kanülenaufnahme aufweist und im Pendelhub zwischen zwei, an unterschiedlichen Seiten des Kanülenmagazins angeordneten Übergabepositionen bewegbar ist. Wird der Entnahmeschlitten in der einen Richtung an dem Kanülenmagazin vorbeibewegt, wird das eine Entnahmefeld mit zumindest einer Kanüle befüllt. Anschließend wird das bereits befüllte Entnahmefeld in die Übergabeposition an der einen Seite des Kanülenmagazins bewegt. Dort wird die zumindest eine Kanüle des befüllten Entnahmefelds bereitgehalten und beispielsweise mit einem Entnahmegreifer gegriffen und einem nachgelagerten Prozessschritt zugeführt. Auf dem Rückweg des Entnahmeschlittens, also beim Rückhub des Entnahmeschlittens, kann das andere Entnahmefeld, das auf der Rückbewegung mit zumindest einer Kanüle befüllt wird oder bereits befüllt wurde, in die andere Übergabeposition bewegt und zumindest eine in dem anderen Entnahmefeld angeordnete Kanüle zur Entnahme, insbesondere mit Hilfe eines Entnahmegreifers, in die Übergabeposition bewegt werden. Auf diese Weise werden Leerhübe des Entnahmeschlittens vermieden, was die Produktivität der Bereitstellungsvorrichtung entsprechend erhöhen kann.

Zur Lösung der Aufgabe wird auch ein Verfahren zur Bereitstellung von Kanülen für einen nachgelagerten Prozessschritt bei der Verarbeitung von Kanülen, insbesondere bei der Herstellung von Spritzen, vorgeschlagen, das die Mittel und Merkmale des unabhängigen, auf ein derartiges Verfahren gerichteten Anspruchs aufweist. Zur Lösung der Aufgabe wird somit ein Verfahren zur Bereitstellung von Kanülen vorgeschlagen, bei dem eine Bereitstellungsvorrichtung nach Anspruch 2 oder nach einem der auf Anspruch 2 rückbezogenen Ansprüche 3 bis 18 verwendet wird, wobei zumindest eine Kanüle mit dem Entnahmeschlitten aus dem Kanülenmagazin der Bereitstellungsvorrichtung entnommen wird, wonach die Lage zumindest einer Kanüle in der wenigstens einen Kanülenaufnahme des Entnahmeschlittens mit Hilfe zumindest eines Sensors erfasst wird, wobei im Falle einer Lageabweichung der Kanüle von einer Soll-Lage die Kanüle mit Hilfe der Lagekorrekturvorrichtung der Bereitstellungsvorrichtung in die Soll-Lage ausgerichtet wird.

Anschließend kann die zumindest eine ordnungsgemäß ausgerichtete Kanüle mit Hilfe eines Entnahmegreifers aus der Kanülenaufnahme des Entnahmeschlittens entnommen und an einen nachgelagerten Prozessschritt übergeben werden.

Zur Lösung der Aufgabe wird schließlich auch ein Computerprogramm vorgeschlagen, das Befehle umfasst, die bewirken, dass die Bereitstellungsvorrichtung nach Anspruch 2 oder nach einem der auf Anspruch 2 rückbezogenen Ansprüche 3 bis 18 das Verfahren nach einem der auf ein solches gerichteten Ansprüche ausführt.

Das Computerprogramm kann auf einem computerlesbaren Medium gespeichert sein. Als computerlesbares Medium kann ein konventioneller Datenträger, wie beispielsweise ein programmierbarer Speicher, ein USB-Speicher, eine CD-ROM oder ein anderes Speichermodul dienen. Selbstverständlich ist als computerlesbares Medium auch ein cloudbasierter Speicher möglich.

Die Erfindung wird nun anhand eines Ausführungsbeispieles näher beschrieben, ist aber nicht auf dieses Ausführungsbeispiel beschränkt.

Es zeigen:
- Fig. 1: eine perspektivische Ansicht einer Bereitstellungsvorrichtung zur Bereitstellung von Kanülen mit einem Kanülenmagazin und einem dem Kanülenmagazin zugeordneten Entnahmeschlitten, an dessen Oberseite insgesamt acht Kanülenaufnahmen ausgebildet sind, in die Kanülen aus dem Kanülenmagazin gelangen, wenn der Entnahmeschlitten in einer Entnahmebewegung an einer unterseitigen Entnahmeöffnung des Kanülenmagazins vorbeibewegt wird,
- Fig. 2: eine perspektivische Darstellung der in Figur 1 dargestellten Bereitstellungsvorrichtung, wobei hier ein Entnahmegreifer der Vorrichtung in Entnahmeposition an dem Entnahmeschlitten angeordnet ist,
- Fig. 3: die in den Figuren 1 und 2 dargestellte Bereitstellungsvorrichtung mit am Ende seiner Entnahmebewegung befindlichem Entnahmeschlitten,
- Fig. 4: eine weitere perspektivische Darstellung der in den Figuren 1 bis 3 gezeigten Bereitstellungsvorrichtung, wobei hier zwei Sicherungsstifte vor die Entnahmeöffnung des Kanülenmagazins geschoben sind, um einen Magazinwechsel vorzubereiten,
- Fig. 5: eine teilausgebrochene, perspektivische Darstellung der in den Figuren 1 bis 4 gezeigten Bereitstellungsvorrichtung zur Veranschaulichung des Anschlusses der Kanülenaufnahmen des Entnahmeschlittens an eine Unterdruckwelle, sowie
- Fig. 6 und 7: perspektivische Ansichten eines Kanülenmagazins zur Veranschaulichung der Funktion der Sicherungsstifte sowie einer Schiebeverbindung zur wechselbaren Befestigung des Kanülenmagazins an einer Halterung der Bereitstellungsvorrichtung.

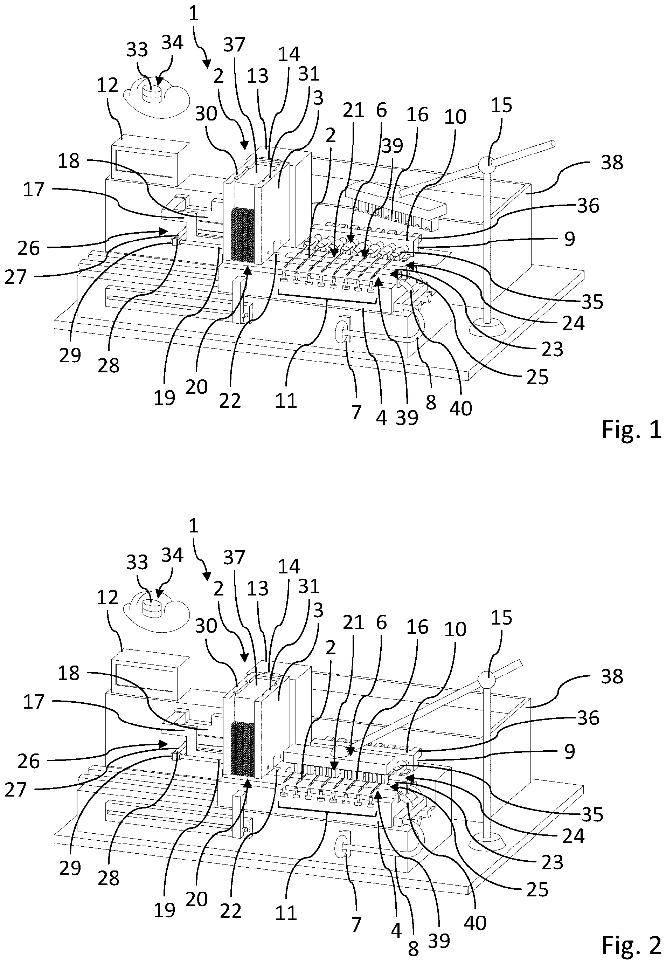

Die Figuren 1 bis 5 zeigen eine im Ganzen mit 1 bezeichnete Bereitstellungsvorrichtung zur Bereitstellung von Kanülen 2 für nachgelagerte Prozessschritte, wie sie beispielsweise bei der Fertigung von Spritzen, für die die Kanülen 2 benötigt werden, angewandt werden.

Die Bereitstellungsvorrichtung 1 weist ein Kanülenmagazin 3 und einen Entnahmeschlitten 4 auf. Der Entnahmeschlitten 4 wird zur Entnahme von Kanülen 2 aus dem Kanülenmagazin 3 in einer Entnahmebewegung an dem Kanülenmagazin 3 vorbeibewegt. Dabei passiert der Entnahmeschlitten 4 eine Entnahmeöffnung 5 des Kanülenmagazins 3, durch die Kanülen 2 dem Entnahmeschlitten 4 aus dem Kanülenmagazin 3 zugeführt werden. Unterstützt durch die Schwerkraft und einen Drücker 37 können die in dem Kanülenmagazin 3 vorgehaltenen Kanülen 2 in die Kanülenaufnahmen 6 des Entnahmeschlittens 4 gelangen. Ferner wird der Entnahmeschlitten 4 dazu verwendet, die Kanülen in eine Übergabeposition zu transportieren und dort für nachgelagerte Bearbeitungsschritte bereitzustellen.

Der Entnahmeschlitten 4 der in den Figuren gezeigten Bereitstellungsvorrichtung 1 weist an seiner Oberseite insgesamt acht Kanülenaufnahmen 6 für jeweils eine Kanüle 2 auf. Die Kanülenaufnahmen 6 sind zur Fixierung der Kanülen 2 in den Kanülenaufnahmen 6 mit einer Unterdruckquelle 7 der Bereitstellungsvorrichtung 1 verbunden. Über eine Unterdruckverbindung 8 wird ein mit der Unterdruckquelle 7 erzeugter Unterdruck auf die Kanülenaufnahmen 6 übertragen und kann dort auf die in den Kanülenaufnahmen 6 positionierten Kanülen 2 wirken, um die Kanülen 2 in den Kanülenaufnahmen 6 zu fixieren.

Die Bereitstellungsvorrichtung 1 weist eine Lagekorrekturvorrichtung 9 auf. Die Lagekorrekturvorrichtung 9 ist dazu eingerichtet, die in den Kanülenaufnahmen 6 angeordneten Kanülen 2 in eine Soll-Lage zu bringen, falls die Kanülen 2 nicht ordnungsgemäß in den Kanülenaufnahmen 6 positioniert sind. Dazu weist die Lagekorrekturvorrichtung 9 eine der Anzahl von Kanülenaufnahmen 6 an dem Entnahmeschlitten 4 entsprechende Anzahl von Ausrichtmitteln 10 auf, mit denen die in den Kanülenaufnahmen 6 positionierten Kanülen 2 geklemmt und ausgerichtet werden können.

Die Lagekorrekturvorrichtung 9 ist durch ihre Ausrichtmittel 10 dazu eingerichtet, die in den Kanülenaufnahmen 6 angeordneten Kanülen 2 um einen definierten Winkelbetrag um ihre Längsachse zu drehen und die Kanülen 2 um eine definierte Strecke axial zu verschieben, um die Kanülen 2 bei Bedarf auszurichten und in ihre Soll-Lage zu bringen. Die Lagekorrekturvorrichtung 9 umfasst insgesamt acht Klemmvorrichtungen 35 und acht mit den Klemmvorrichtungen 35 verbundene Antriebe 36. Mit den Klemmvorrichtungen 35 können in den Kanülenaufnahmen 6 angeordnete Kanülen 2 geklemmt und bei Feststellung einer Lageabweichung mithilfe der Antriebe 36 gedreht und/oder axial verschoben werden, um die Lageabweichung zu beheben.

Die Bereitstellungsvorrichtung 1 weist mehrere Sensoren 11 auf, die bei dem in den Figuren gezeigten Ausführungsbeispiel der Bereitstellungsvorrichtung 1 als Kameras ausgebildet sind. Die Sensoren 11 dienen zur Feststellung, ob die Kanülenaufnahmen 6 mit Kanülen 2 belegt sind und ferner, ob die in den Kanülenaufnahmen 6 angeordneten Kanülen 2 ordnungsgemäß ausgerichtet sind.

Lässt sich eine Lageabweichung einzelner oder mehrerer oder sämtlicher Kanülen 2 in den Kanülenaufnahmen 6 von einer Soll-Lage feststellen, kann die Lage der Kanülen 2 in den Kanülenaufnahmen 6 des Entnahmeschlittens 4 mit Hilfe der Lagekorrekturvorrichtung 9 korrigiert werden. Die Lage kann hierbei sowohl eine Position der Kanüle 2 in der Kanülenaufnahme 6 als auch eine Drehstellung der Kanüle 2 in der Kanülenaufnahme 6 umfassen.

Hierfür ist die Bereitstellungsvorrichtung 1 mit einer Steuereinheit 12 ausgestattet. Die Steuereinheit 12 ist dazu eingerichtet, die Lagekorrekturvorrichtung 9 in Abhängigkeit eines von dem wenigstens einen Sensor 11 übermittelten Sensorsignals anzusteuern, um eine oder mehrere der in den Kanülenaufnahmen 6 angeordneten Kanülen 2 in auszurichten, falls eine Lageabweichung mit Hilfe der Sensoren 11 festgestellt werden sollte.

Das in den Figuren gezeigte Kanülenmagazin 3 der Bereitstellungsvorrichtung ist als Wechselmagazin ausgebildet. Die Bereitstellungsvorrichtung 1 weist eine Halterung 13 auf, an der das als Wechselmagazin ausgebildete Kanülenmagazin 3 wechselbar befestigt ist. Durch die Halterung 13 und die Ausbildung des Kanülenmagazins 3 als Wechselmagazin lässt sich das Kanülenmagazin 3 bei Bedarf sehr einfach und vor allem schnell gegen ein mit anderen Kanülen 2 gefülltes Kanülenmagazin 3 austauschen.

Das Kanülenmagazin 3 ist über eine lösbare formschlüssige Verbindung 14, die hier als Schiebeverbindung, nämlich als Schwalbenschwanzverbindung ausgebildet ist, mit der Halterung 13 verbunden.

Die Bereitstellungsvorrichtung 1 weist einen Entnahmegreifer 15 auf. Der Entnahmegreifer 15 ist als Unterdruckgreifer ausgebildet und seinerseits ebenfalls mit einer Unterdruckquelle 7 verbunden.

Der Entnahmegreifer 15 weist insgesamt acht und somit eine Anzahl an Greifaufnahmen 16 auf, die der Anzahl an Kanülenaufnahmen 6 an dem Entnahmeschlitten 4 entspricht.

Die Bereitstellungsvorrichtung 1 weist ferner ein Hubmittel 17 in Form einer Hubleiste auf. Das Hubmittel 17 dient dazu, einen in dem Kanülenmagazin 3 befindlichen Kanülenvorrat bei Bedarf anzuheben und abzusenken. Eine Bewegung des Hubmittels 17 ist dabei durch eine Führung 18 vorgegeben und geführt.

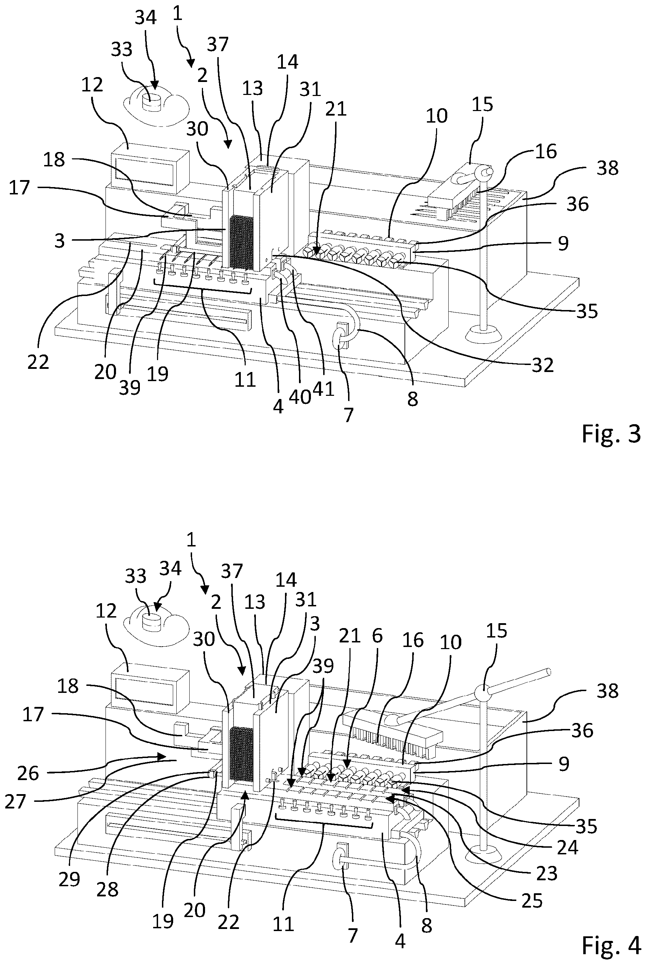

Die Funktion des Hubmittels 17 wird bei Betrachtung von Figur 4 deutlich. Figur 4 zeigt die Bereitstellungsvorrichtung 1 nachdem ein neues Kanülenmagazin 3 an der Halterung 13 angebracht wurde. Zu erkennen sind zwei Sicherungsmittel 19 in Form von Sicherungsstiften, durch die die Entnahmeöffnung 5 des Kanülenmagazins 3 unterseitig verschlossen ist. Durch die Sicherungsmittel 19 werden die in dem Kanülenmagazin 3 vorgehaltenen Kanülen 2 zurückgehalten. Bevor die Sicherungsmittel 19 gezogen werden können, um die Entnahmeöffnung 5 des Kanülenmagazins 3 für die Kanülen 2 passierbar zu machen, wird das Hubmittel 17 in die in Figur 4 gezeigte Position gebracht. Dazu wird das Hubmittel 17 von unten in die Entnahmeöffnung 15 des Kanülenmagazins 3 geschoben, wodurch die in dem Kanülenmagazin 3 angeordneten Kanülen 2 angehoben werden. Anschließend können die Sicherungsmittel 19 gezogen werden, um die Entnahmeöffnung 5 des Kanülenmagazins 3 für die Kanülen 2 zu öffnen.

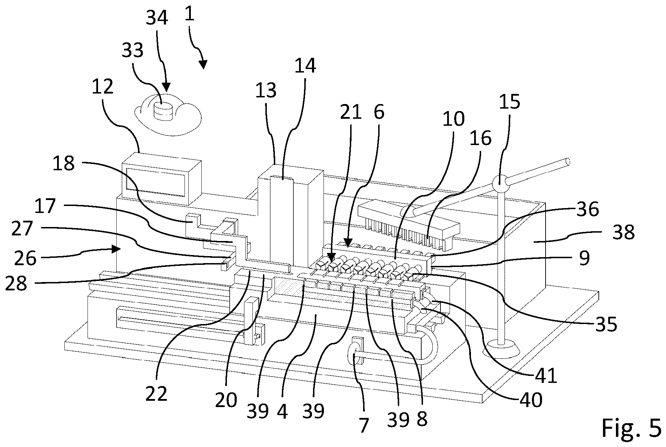

Bevor die eigentliche Entnahme der Kanülen 2 aus dem Kanülenmagazin 3 mit Hilfe des Entnahmeschlittens erfolgen kann, setzt das Hubmittel 17 die Kanülen 2 vorsichtig auf der Oberseite des Entnahmeschlittens 4 ab. Dabei gelangen die Kanülen 2 in Kontakt mit einem Bereich 20 des Entnahmeschlittens 4, der benachbart zu einem Entnahmefeld 21 mit den Kanülenaufnahmen 6 an der Oberseite des Entnahmeschlittens angeordnet ist. Damit das Hubmittel 17 die Kanülen 2 auf den Bereich 20 des Entnahmeschlittens 4 ablegen kann, weist der Entnahmeschlitten 4 eine randseitig offene Längsnut 22 auf, in die das Hubmittel 17 bei unterhalb des Kanülenmagazins 3 angeordnetem Entnahmeschlitten 4 eintauchen kann. Figur 5 zeigt das Hubmittel 17 oberhalb der Längsnut 22 in dem Entnahmeschlitten 4.

Der Entnahmeschlitten 4 weist ferner eine Aussparung 23 auf, in die der zuvor bereits erwähnte Entnahmegreifer 15 bei der Entnahme von Kanülen 2 aus den Kanülenaufnahmen 6 bewegt werden kann. Die Aussparung 23 verläuft rechtwinklig zu den insgesamt acht Kanülenaufnahmen 6. Jede der Kanülenaufnahmen 6 wird durch die Aussparung 23 in zwei Aufnahmeabschnitte 24 und 25 unterteilt. Dabei sind die Aufnahmeabschnitte 24 und 25 über die beispielsweise in Figur 5 gut zu erkennende Unterdruckverbindung 8 mit der Unterdruckquelle 7 verbunden. Wie bereits zuvor erwähnt, ist das Hubmittel 17 durch die Entnahmeöffnung 5 des Kanülenmagazins 3 in das Kanülenmagazin 3 einführbar, um den Kanülenvorrat in dem Kanülenmagazin 3 in der zuvor beschriebenen Art und Weise anzuheben und bei Bedarf auch wieder abzusenken.

Die Figuren 1 bis 5 verdeutlichen, dass jede Kanülenaufnahme 6 des Entnahmeschlittens 4 jeweils zwei mit der Unterdruckquelle 7 verbundene Ansaugstellen 39 aufweist. Über die Ansaugstellen 39 kann der von der Unterdruckquelle 7 bereitgestellte Unterdruck zur Fixierung der Kanülen 2 auf die Kanülenaufnahmen 6 und die darin positionierten Kanülen 2 übertragen werden. Wie bereits zuvor ausgeführt, ist jede der Kanülenaufnahmen 6 des in den Figuren gezeigten Entnahmeschlittens 4 in zwei Aufnahmeabschnitte 24 und 25 unterteilt. Jeder der Aufnahmeabschnitte 24 und 25 einer jeden Kanülenaufnahme 6 weist jeweils zumindest eine mit der Unterdruckquelle 7 verbundene Ansaugstelle 39 auf. Auf diese Weise lassen sich die Kanülen 2 besonders zuverlässig mittels Unterdrucks in den Aufnahmeabschnitten 24 und 25 der einzelnen Kanülenaufnahmen 6 des Entnahmeschlittens 4 fixieren. Die beiden Reihen von Aufnahmeabschnitten 24 und 25 der Kanülenaufnahmen 6 des Entnahmeschlittens 4 sind über jeweils eine Unterdruckleitung 40 beziehungsweise 41, die jeweils Teil der zuvor bereits erwähnten Unterdruckverbindung 8 ist, an die Unterdruckquelle 7 angeschlossen.

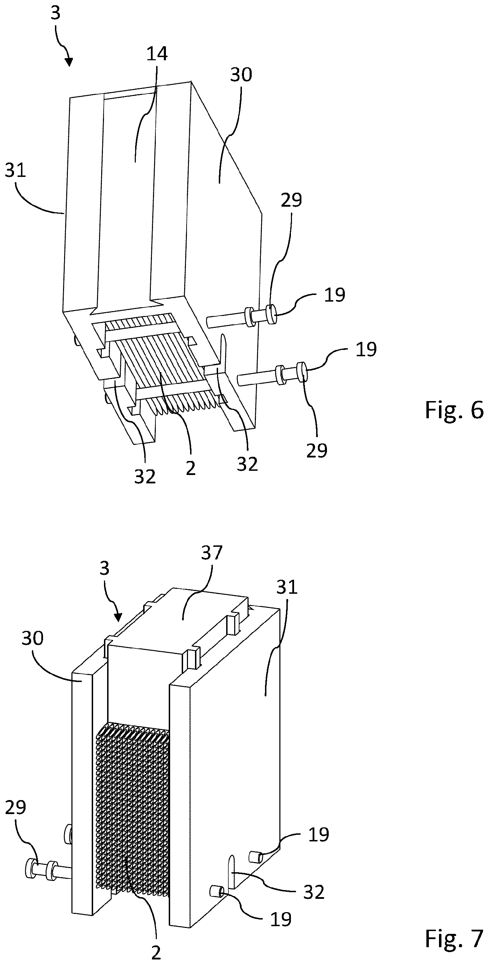

Zum Entfernen und Anbringen der beiden Sicherungsmittel 19 weist die Bereitstellungsvorrichtung 1 eine Vorrichtung 26 zum Entfernen und Anbringen der Sicherungsmittel 19 auf. Die Vorrichtung 26 umfasst eine Leiste 27 mit zwei voneinander beabstandeten Nuten 28, in die Köpfe 29 der Sicherungsmittel 19 eingreifen können. Die Köpfe 29 der Sicherungsmittel 19 sind beispielsweise gut in den Figuren 6 und 7 zu erkennen. In den Nuten 28, von denen eine beispielsweise in Figur 5 zu erkennen ist, lassen sich die Köpfe 29 der Sicherungsmittel 19 in Bezug auf eine Steckrichtung und eine Auszugsrichtung der Sicherungsmittel 19 aus ihrer Position an dem Kanülenmagazin 3 formschlüssig festlegen, so dass sie mit Hilfe der Leiste 27 der Vorrichtung 26 gesteckt und auch gezogen werden können.

Insbesondere Figur 6 verdeutlicht, dass das Kanülenmagazin 3 an zwei sich gegenüberliegenden Wänden 30, 31 jeweils einen Schlitz 32 aufweist, durch den das Hubmittel 17 zum Anheben und Absenken der Kanülen 2 in dem Kanülenmagazin 3 in das Kanülenmagazin 3 eingeführt werden kann.

Bei einer in den Figuren nicht gezeigten Ausführungsform der Bereitstellungsvorrichtung 1 weist der Entnahmeschlitten 3 zwei Entnahmefelder mit jeweils zumindest einer Kanülenaufnahme 6 für eine Kanüle 2 auf. Der Entnahmeschlitten 3 dieser Ausführungsform der Bereitstellungsvorrichtung 1 kann im Pendelhub zwischen zwei, an unterschiedlichen Seiten des Kanülenmagazins 3 angeordneten Übergabepositionen bewegt werden. Auf diese Weise wird ein Leerhub des Entnahmeschlittens 4 vermieden, was die Produktivität der Bereitstellungsvorrichtung 1 steigern kann.

Unter Verwendung der in den Figuren gezeigten Bereitstellungsvorrichtung 1 lässt sich nachfolgendes Verfahren zur Bereitstellung von Kanülen 2 für einen nachgelagerten Prozessschritt bei der Verarbeitung von Kanülen 2, insbesondere bei der Herstellung von Spritzen, durchführen. Dabei ist vorgesehen, dass die Kanülen 2 mit dem Entnahmeschlitten 4 aus dem Kanülenmagazin 3 der Bereitstellungsvorrichtung 1 entnommen werden. Anschließend wird die Lage zumindest einer Kanüle 2 in wenigstens einer der Kanülenaufnahmen 6 des Entnahmeschlittens 4 mit Hilfe eines Sensors 11 erfasst. Anschließend kann eine Kanüle 2, die eine Lageabweichung aufweist, mit Hilfe der Lagekorrekturvorrichtung 9 der Bereitstellungsvorrichtung 1 in Abhängigkeit einer mit Hilfe des Sensors 11 und der Steuereinheit 12 der Bereitstellungsvorrichtung erfassten Lageabweichung der Kanüle 2 in eine Soll-Lage gebracht werden.

Anschließend können die Kanülen 2 in ihrer bestimmungsgemäßen Lage mit Hilfe des Entnahmegreifers 15 aus den Kanülenaufnahmen 6 des Entnahmeschlittens 4 entnommen und an einen nachgelagerten Prozessschritt übergeben werden. Die Übergabe der Kanülen 2 an eine nachgelagerte Bearbeitungsvorrichtung 38 zur Durchführung eines nachgelagerten Prozessschritts ist beispielsweise in Figur 3 dargestellt. In einem der nachgelagerten Prozessschritte ist es möglich, die Kanülen 2 einer Spritzgießform zuzuführen und mit Kunststoff zu umspritzen, um beispielsweise Spritzen herzustellen.

Auf einem computerlesbaren Medium 33, hier in einem cloudbasierten Datenspeicher, ist ein Computerprogramm 34 gespeichert, das Befehle umfasst, die bewirken, dass die Bereitstellungsvorrichtung 1das zuvor beschriebene Verfahren ausführt.

Die Erfindung befasst sich mit Verbesserungen auf dem technischen Gebiet der Bereitstellung von Kanülen 2. Hierzu wird unter anderem eine Bereitstellungsvorrichtung 1 vorgeschlagen, deren Entnahmeschlitten 4 zumindest eine Kanülenaufnahme 6 zur Aufnahme einer Kanüle 2 aufweist, die zur Fixierung einer Kanüle 2 in der Kanülenaufnahme 6 mit einer Unterdruckquelle 7 verbindbar oder verbunden ist.

### Bezugszeichenliste

- 1: Bereitstellungsvorrichtung
- 2: Kanüle
- 3: Kanülenmagazin
- 4: Entnahmeschlitten
- 5: Entnahmeöffnung
- 6: Kanülenaufnahme
- 7: Unterdruckquelle
- 8: Unterdruckverbindung
- 9: Lagekorrekturvorrichtung
- 10: Ausrichtmittel
- 11: Sensor
- 12: Steuereinheit
- 13: Halterung
- 14: lösbare formschlüssige Verbindung
- 15: Entnahmegreifer
- 16: Greifaufnahmen
- 17: Hubmittel
- 18: Führung
- 19: Sicherungsmittel
- 20: Bereich
- 21: Entnahmefeld
- 22: Längsnut
- 23: Aussparung
- 24: Aufnahmeabschnitt
- 25: Aufnahmeabschnitt
- 26: Vorrichtung zum Entfernen und/oder Anbringen der Sicherungsmittel
- 27: Leiste
- 28: Nut
- 29: Köpfe von 19
- 30: Wand von 3
- 31: Wand von 3
- 32: Schlitz
- 33: computerlesbares Medium, cloud-basierter Datenspeicher
- 34: Computerprogramm
- 35: Klemmvorrichtung
- 36: Antrieb
- 37: Drücker
- 38: nachgelagerte Bearbeitungsvorrichtung
- 39: Ansaugstelle in 6,24,25
- 40: Unterdruckleitung
- 41: Unterdruckleitung

## Patentansprüche

1. Bereitstellungsvorrichtung (1) zur Bereitstellung von Kanülen (2) mit einem Kanülenmagazin (3) und mit einem Entnahmeschlitten (4) zur Entnahme von Kanülen (2) aus dem Kanülenmagazin (3) und zum Transport der Kanülen (2) in eine Bereitstellungsposition, wobei der Entnahmeschlitten (4) zumindest eine Kanülenaufnahme (6) für eine Kanüle (2) aufweist, die zur Fixierung einer Kanüle (2) in der Entnahmeschlitten (4) mit einer Unterdruckquelle (7) verbindbar oder verbunden ist, **dadurch gekennzeichnet, dass** die Bereitstellungsvorrichtung (1) ein Hubmittel (17) zum Anheben und Absenken eines in dem Kanülenmagazin (3) befindlichen Kanülenvorrats aufweist, und dass der Entnahmeschlitten (4) eine Längsnut (22) aufweist, in die das Hubmittel (17) eintauchen kann.

2. Bereitstellungsvorrichtung (1) nach dem vorherigen Anspruch, wobei die Bereitstellungsvorrichtung (1) eine Lagekorrekturvorrichtung (9) aufweist, die dazu eingerichtet ist, eine in der zumindest einen Kanülenaufnahme (6) angeordnete Kanüle (2) in eine Soll-Lage auszurichten.

3. Bereitstellungsvorrichtung (1) nach dem vorherigen Anspruch, wobei die Lagekorrekturvorrichtung (9) dazu eingerichtet ist, eine in der zumindest einen Kanülenaufnahme (6) angeordnete Kanüle (2), insbesondere um ihre Längsachse, um einen definierten Winkelbetrag zu drehen und/oder die Kanüle (2) um eine definierte Strecke axial zu verschieben, um die Kanüle (2) in eine Soll-Lage auszurichten, insbesondere wobei die Lagekorrekturvorrichtung (9) zumindest ein Ausrichtmittel (10) zur Veränderung der Lage einer in der zumindest einen Kanülenaufnahme (6) angeordneten Kanüle (2) aufweist, insbesondere zumindest eine Klemmvorrichtung (35) und/oder zumindest einen Antrieb (36) umfassen.

4. Bereitstellungsvorrichtung (1) nach einem der vorherigen Ansprüche, wobei die Bereitstellungsvorrichtung (1) wenigstens einen Sensor (11), insbesondere eine Kamera, zur Kontrolle der Anwesenheit und/oder Lage einer in der zumindest einen Kanülenaufnahme (6) des Entnahmeschlittens (4) angeordneten Kanüle aufweist.

5. Bereitstellungsvorrichtung (1) nach Anspruch 4 soweit rückbezogen auf Anspruch 2, wobei die Bereitstellungsvorrichtung (1) eine Steuereinheit (12) aufweist, die dazu eingerichtet ist, die Lagekorrekturvorrichtung (9) in Abhängigkeit eines von dem wenigstens einen Sensor (11) übermittelten Sensorsignals anzusteuern, um eine in der wenigstens einen Kanülenaufnahme (6) angeordnete Kanüle (2) in ihre Soll-Lage auszurichten.

6. Bereitstellungsvorrichtung (1) nach einem der vorherigen Ansprüche, wobei der Entnahmeschlitten (4) zur Entnahme von Kanülen (2) aus dem Kanülenmagazin (3) in einer Entnahmebewegung an dem Kanülenmagazin (3) vorbei bewegbar ist, wobei das Kanülenmagazin (3) als Wechselmagazin ausgebildet ist, insbesondere wobei die Bereitstellungsvorrichtung (1) eine Halterung (13) aufweist, an der das Kanülenmagazin (3) wechselbar befestigt ist.

7. Bereitstellungsvorrichtung (1) nach einem der vorherigen Ansprüche, wobei das Kanülenmagazin (3) über eine lösbare formschlüssige Verbindung (14), insbesondere über eine Schiebeverbindung, zum Beispiel eine Schwalbenschwanzverbindung, mit einer Halterung (13) der Bereitstellungsvorrichtung (1) verbunden ist.

8. Bereitstellungsvorrichtung (1) nach einem der vorherigen Ansprüche, wobei die Bereitstellungsvorrichtung (1) einen Entnahmegreifer (15), insbesondere einen Unterdruckgreifer, aufweist.

9. Bereitstellungsvorrichtung (1) nach dem vorherigen Anspruch, wobei der Entnahmegreifer (15) eine der Anzahl der Kanülenaufnahmen (6) an dem Entnahmeschlitten (4) entsprechende Anzahl von Greifaufnahmen (16) aufweist, vorzugsweise die Greifaufnahme/die Greifaufnahmen (16) mit einer Unterdruckquelle (7) verbunden sind.

10. Bereitstellungsvorrichtung (1) nach einem der vorherigen Ansprüche, wobei die Bereitstellungsvorrichtung (1) als Hubmittel (17) zum Anheben und Absenken eines in dem Kanülenmagazin (3) befindlichen Kanülenvorrats eine Hubleiste aufweist.

11. Bereitstellungsvorrichtung nach einem der vorherigen Ansprüche, wobei eine Bewegung des Hubmittels (17) durch eine Führung (18) geführt ist.

12. Bereitstellungsvorrichtung (1) nach einem der vorherigen Ansprüche, wobei die Längsnut des Entnahmeschlittens (4) eine randseitig offene Längsnut (22) ist, und/oder wobei das Hubmittel (17) bei unterhalb des Kanülenmagazins (3) angeordnetem Entnahmeschlitten (4), in die Längsnut (22) eintauchen kann.

13. Bereitstellungsvorrichtung (1) nach einem der vorherigen Ansprüche, wobei der Entnahmeschlitten (4) eine Aussparung (23) aufweist, in die ein oder der Entnahmegreifer (15) bei der Entnahme von Kanülen (2) bewegbar ist, insbesondere wobei die Aussparung (23) quer oder rechtwinklig zu der zumindest einen Kanülenaufnahme (6) ausgerichtet ist und/oder die zumindest eine Kanülenaufnahme (6) in zwei Aufnahmeabschnitte (24, 25) unterteilt, vorzugsweise die jeweils für sich mit einer oder der Unterdruckquelle (7) verbunden sind.

14. Bereitstellungsvorrichtung (1) nach einem der vorherigen Ansprüche, wobei in oder an der zumindest einen Kanülenaufnahme (6), insbesondere in oder an jeder Kanülenaufnahme (6) jeweils, zumindest eine mit der Unterdruckquelle (7) verbundene Ansaugstelle (39) vorhanden ist, vorzugsweise wobei in oder an jedem Aufnahmeabschnitt (24,25) der zumindest einen Kanülenaufnahme (6) jeweils zumindest eine mit der Unterdruckquelle (7) verbundene Ansaugstelle (39) vorhanden ist.

15. Bereitstellungsvorrichtung (1) nach einem der vorherigen Ansprüche, wobei das Hubmittel (17) durch eine Entnahmeöffnung (5) des Kanülenmagazin (3) in das Kanülenmagazin (3) einführbar ist, um einen Kanülenvorrat in dem Kanülenmagazin (3) anzuheben.

16. Bereitstellungsvorrichtung (1) nach einem der vorherigen Ansprüche, wobei das Kanülenmagazin (3) zumindest ein Sicherungsmittel (19), insbesondere einen Sicherungsstift aufweist, mit dem eine Entnahmeöffnung (5) des Kanülenmagazin (3) verschließbar ist.

17. Bereitstellungsvorrichtung (1) nach einem der vorherigen Ansprüche, wobei die Bereitstellungsvorrichtung (1) eine Vorrichtung (26) zum Entfernen und/oder Anbringen des zumindest einen Sicherungsmittels (19) aufweist.

18. Bereitstellungsvorrichtung (1) nach einem der vorherigen Ansprüche, wobei der Entnahmeschlitten (3) zwei Entnahmefelder mit jeweils zumindest einer Kanülenaufnahme (6) für eine Kanüle (2) aufweist und im Pendelhub zwischen zwei, an unterschiedlichen Seiten des Kanülenmagazins (3) angeordneten Übergabepositionen bewegbar ist.

19. Verfahren zur Bereitstellung von Kanülen (2) für einen nachgelagerten Prozessschritt bei der Verarbeitung von Kanülen (2), insbesondere bei der Herstellung von Spritzen, unter Verwendung einer Bereitstellungsvorrichtung (1) nach Anspruch 2 oder nach einem der auf Anspruch 2 rückbezogenen Ansprüche 3 bis 18, wobei die Kanülen (2) mit dem Entnahmeschlitten (4) aus dem Kanülenmagazin (3) der Bereitstellungsvorrichtung (1) entnommen werden, wonach die Lage zumindest einer Kanüle (2) in der wenigstens einen Kanülenaufnahme (6) des Entnahmeschlittens (4) mithilfe zumindest eines Sensors (11) erfasst wird und wonach die Kanüle (2) mithilfe der Lagekorrekturvorrichtung (9) der Bereitstellungsvorrichtung (1) in Abhängigkeit einer mithilfe des Sensors (11) und einer Steuereinheit (12) der Bereitstellungsvorrichtung (1) erfassten Lageabweichung der Kanüle (2) von einer Soll-Lage in die Ist-Lage gebracht wird.

20. Verfahren nach dem vorherigen Anspruch, wobei die zumindest eine Kanüle (2) mithilfe eines Entnahmegreifers (15) aus der Kanülenaufnahme (6) des Entnahmeschlittens (4) entnommen und an einen nachgelagerten Prozessschritt übergeben wird.

21. Computerprogramm (34), umfassend Befehle, die bewirken, dass die Bereitstellungsvorrichtung (1) nach Anspruch 2 oder nach einem der auf Anspruch 2 rückbezogenen Ansprüche 3 bis 18 das Verfahren nach einem der Ansprüche 19 oder 20 ausführt, wenn das Programm durch einen die Bereitstellungsvorrichtung (1) steuernden Rechner durchgeführt wird.

22. Computerlesbares Medium (33), auf dem das Computerprogramm nach dem vorherigen Anspruch gespeichert ist.

## Claims

1. Supply device (1) for supplying cannulas (2), having a cannula magazine (3) and having a retrieving slide (4) for retrieving cannulas (2) from the cannula magazine (3) and for transporting the cannulas (2) to a supply position, wherein the retrieving slide (4) has at least one cannula receptacle (6) for a cannula (2), which for fixing a cannula (2) in the retrieving slide (4) is connectable, or connected, to a vacuum source (7),
**characterized in that** the supply device (1) has a lifting means (17) for lifting and lowering a cannula reserve located in the cannula magazine (3), and **in that** the retrieving slide (4) has a longitudinal groove (22) into which the lifting means (17) can plunge.

2. Supply device (1) according to the preceding claim, wherein the supply device (1) has a position-correction device (9) which is specified to align a cannula (2) disposed in the at least one cannula receptacle (6) in a target position.

3. Supply device (1) according to the preceding claim, wherein the position-correction device (9) is specified to rotate a cannula (2) disposed in the at least one cannula receptacle (6) by a defined angular value, in particular about the longitudinal axis of said cannula (2), and/or to displace the cannula (2) axially by a defined distance, so as to align the cannula (2) in a target position, in particular wherein the position-correction device (9) has at least one aligning means (10), in particular comprising at least one clamping device (35) and/or at least one drive (36), for altering the position of a cannula (2) disposed in the at least one cannula receptacle (6).

4. Supply device (1) according to one of the preceding claims, wherein the supply device (1) has at least one sensor (11), in particular a camera, for checking the presence and/or position of a cannula disposed in the at least one cannula receptacle (6) of the retrieving slide (4).

5. Supply device (1) according to Claim 4 if referring back to Claim 2, wherein the supply device (1) has a control unit (12) which is specified to actuate the position-correction device (9) as a function of a sensor signal transmitted by the at least one sensor (11), so as to align a cannula (2) disposed in the at least one cannula receptacle (6) in the target position of said cannula (2).

6. Supply device (1) according to one of the preceding claims, wherein the retrieving slide (4) for retrieving cannulas (2) from the cannula magazine (3) is able to be moved past the cannula magazine (3) in a retrieving movement, wherein the cannula magazine (3) is designed as an interchangeable magazine, in particular wherein the supply device (1) has a mounting (13) to which the cannula magazine (3) is interchangeably fastened.

7. Supply device (1) according to one of the preceding claims, wherein the cannula magazine (3) is connected to a mounting (13) of the supply device (1) by way of a releasable form-fitting connection (14), in particular by way of a sliding connection, for example a dovetail connection.

8. Supply device (1) according to one of the preceding claims, wherein the supply device (1) has a retrieving gripper (15), in particular a vacuum gripper.

9. Supply device (1) according to the preceding claim, wherein the retrieving gripper (15) has a number of gripper receptacles (16) corresponding to the number of cannula receptacles (6) on the retrieving slide (4), the gripper receptacle/the gripper receptacles (16) preferably being connected to a vacuum source (7) .

10. Supply device (1) according to one of the preceding claims, wherein the supply device (1) as the lifting means (17) has a lifting strip for lifting and lowering a cannula reserve located in the cannula magazine (3).

11. Supply device according to one of the preceding claims, wherein a movement of the lifting means (17) is guided by a guide (18).

12. Supply device (1) according to one of the preceding claims, wherein the longitudinal groove of the retrieving slide (4) is a peripherally open longitudinal groove (22), and/or wherein the lifting means (17) can plunge into the longitudinal groove (22) when the retrieving slide (4) is disposed below the cannula magazine (3).

13. Supply device (1) according to one of the preceding claims, wherein the retrieving slide (4) has a clearance (23) into which a or the retrieving gripper (15) is able to be moved when retrieving cannulas (2), in particular wherein the clearance (23) is aligned transversely or orthogonally to the at least one cannula receptacle (6) and/or divides the at least one cannula receptacle (6) into two receptacle portions (24, 25), each of the latter preferably being connected individually to a or the vacuum source (7).

14. Supply device (1) according to one of the preceding claims, wherein at least one suction point (39), which is connected to the vacuum source (7), is in each case present in or on the at least one cannula receptacle (6), in particular in or on each cannula receptacle (6), preferably wherein at least one suction point (39) connected to the vacuum source (7) is in each case present in or on each receptacle portion (24, 25) of the at least one cannula receptacle (6).

15. Supply device (1) according to one of the preceding claims, wherein the lifting means (17) is able to be introduced into the cannula magazine (3) through a retrieval opening (5) of the cannula magazine (3), so as to lift a cannula reserve in the cannula magazine (3).

16. Supply device (1) according to one of the preceding claims, wherein the cannula magazine (3) has at least one securing means (19), in particular a securing pin, by way of which a retrieval opening (5) of the cannula magazine (3) is able to be locked.

17. Supply device (1) according to one of the preceding claims, wherein the supply device (1) has a device (26) for removing and/or attaching the at least one securing means (19).

18. Supply device (1) according to one of the preceding claims, wherein the retrieving slide (3) has two retrieval fields having in each case at least one cannula receptacle (6) for a cannula (2), and is able to be moved so as to shuttle between two transfer positions that are disposed on different sides of the cannula magazine (3).

19. Method for supplying cannulas (2) for a downstream process step in the processing of cannulas (2), in particular in the production of syringes, while using a supply device (1) according to Claim 2, or according to one of Claims 3 to 18 referring back to Claim 2, wherein the cannulas (2) are retrieved from the cannula magazine (3) of the supply device (1) by the retrieving slide (4), whereupon the position of at least one cannula (2) in the at least one cannula receptacle (6) of the retrieving slide (4) is detected with the aid of at least one sensor (11), and whereupon the cannula (2), with the aid of the position-correction device (9) of the supply device (1), is moved to the actual position as a function of a positional deviation of the cannula (2) from a target position detected with the aid of the sensor (11) and a control unit (12) of the supply device (1).

20. Method according to the preceding claim, wherein the at least one cannula (2) is retrieved from the cannula receptacle (6) of the retrieving slide (4) with the aid of a retrieving gripper (15) and transferred to a downstream process step.

21. Computer program (34), comprising commands which have the effect that the supply device (1) according to Claim 2, or according to one of Claims 3 to 18 referring back to Claim 2, carries out the method according to one of Claims 19 or 20, when the program is carried out by a computer which controls the supply device (1).

22. Computer-readable medium (33) on which the computer program according to the preceding claim is stored.

## Revendications

1. Dispositif de fourniture (1) pour la fourniture de canules (2) comprenant un magasin de canules (3) et un chariot de prélèvement (4) pour prélever des canules (2) à partir du magasin de canules (3) et pour transporter les canules (2) dans une position de fourniture, dans lequel le chariot de prélèvement (4) présente au moins un logement de canule (6) pour une canule (2), qui peut être connecté ou qui est connecté à une source de vide (7) pour fixer une canule (2) dans le chariot de prélèvement (4), **caractérisé en ce que** le dispositif de fourniture (1) est équipé d'un moyen de levage (17) pour soulever et abaisser une réserve de canules se trouvant à l'intérieur du magasin de canules (3), et **en ce que** le chariot de prélèvement (4) présente une rainure longitudinale (22) dans laquelle le moyen de levage (17) peut s'enfoncer.

2. Dispositif de fourniture (1) selon la revendication précédente, dans lequel le dispositif de fourniture (1) comprend un dispositif de correction de position (9) qui est agencé de manière à orienter une canule (2) agencée à l'intérieur dudit au moins un logement de canule (6) en direction d'une position de consigne.

3. Dispositif de fourniture (1) selon la revendication précédente, dans lequel le dispositif de correction de position (9) est conçu de manière à faire tourner une canule (2) agencée à l'intérieur dudit au moins un logement de canule (6), en particulier autour de son axe longitudinal, d'une valeur angulaire définie et/ou pour déplacer la canule (2) axialement sur une distance définie, afin d'orienter la canule (2) dans une position de consigne, dans lequel le dispositif de correction de position (9) comprend en particulier au moins un moyen d'orientation (10) pour modifier la position d'une canule (2) agencée à l'intérieur dudit au moins un logement de canule (6), comprenant en particulier au moins un dispositif de serrage (35) et/ou au moins un mécanisme d'entraînement (36).

4. Dispositif de fourniture (1) selon l'une quelconque des revendications précédentes, dans lequel le dispositif de fourniture (1) comprend au moins un capteur (11), en particulier une caméra, pour contrôler la présence et/ou la position d'une canule agencée à l'intérieur dudit au moins un logement de canule (6) du chariot de prélèvement (4).

5. Dispositif de fourniture (1) selon la revendication 4 en se référant à la revendication 2, dans lequel le dispositif de fourniture (1) comprend une unité de commande (12) conçue de manière à commander le dispositif de correction de position (9) en fonction d'un signal de capteur transmis par ledit au moins un capteur (11), afin d'orienter une canule (2) agencée à l'intérieur dudit au moins un logement de canule (6) en direction de sa position de consigne.

6. Dispositif de fourniture (1) selon l'une quelconque des revendications précédentes, dans lequel le chariot de prélèvement (4) est mobile de manière à prélever des canules (2) à l'intérieur du magasin de canules (3) en passant devant le magasin de canules (3) dans un mouvement de prélèvement, dans lequel le magasin de canules (3) est conçu sous la forme d'un magasin interchangeable, et dans lequel le dispositif de fourniture (1) comprend en particulier un support (13) sur lequel le magasin de canules (3) est fixé de façon interchangeable.

7. Dispositif de fourniture (1) selon l'une quelconque des revendications précédentes, dans lequel le magasin de canules (3) est connecté à un support (13) du dispositif de fourniture (1) par l'intermédiaire d'une connexion amovible à complémentarité de forme (14), en particulier par l'intermédiaire d'une connexion coulissante, par exemple une connexion en queue d'aronde.

8. Dispositif de fourniture (1) selon l'une quelconque des revendications précédentes, dans lequel le dispositif de fourniture (1) comprend une pince de prélèvement (15), en particulier une pince à vide.

9. Dispositif de fourniture (1) selon la revendication précédente, dans lequel la pince de prélèvement (15) présente un certain nombre de logements de préhension (16) qui correspond au nombre de logements de canule (6) sur le chariot de prélèvement (4), dans lequel le ou les logement(s) de préhension (16) est/sont de préférence connecté(s) à une source de vide (7).

10. Dispositif de fourniture (1) selon l'une quelconque des revendications précédentes, dans lequel le dispositif de fourniture (1) comprend une barre de levage faisant office de moyen de levage (17) pour soulever et abaisser une réserve de canules se trouvant à l'intérieur du magasin de canules (3).

11. Dispositif de fourniture selon l'une quelconque des revendications précédentes, dans lequel un mouvement du moyen de levage (17) est guidé par l'intermédiaire d'un guide (18).

12. Dispositif de fourniture (1) selon l'une quelconque des revendications précédentes, dans lequel la rainure longitudinale du chariot de prélèvement (4) est une rainure longitudinale ouverte sur les bords (22), et/ou dans lequel le moyen de levage (17) peut s'enfoncer à l'intérieur de la rainure longitudinale (22) lorsque le chariot de prélèvement (4) est positionné en dessous du magasin de canules (3).

13. Dispositif de fourniture (1) selon l'une quelconque des revendications précédentes, dans lequel le chariot de prélèvement (4) présente un évidement (23) dans lequel une ou les pince(s) de prélèvement (15) est/sont mobile(s) lors du prélèvement des canules (2), et dans lequel l'évidement (23) est en particulier orienté transversalement ou perpendiculairement audit au moins un logement de canule (6) et/ou divise ledit au moins un logement de canule (6) en deux sections de logement (24, 25) qui sont de préférence connectées séparément à une ou à la source de vide (7).

14. Dispositif de fourniture (1) selon l'une quelconque des revendications précédentes, dans lequel au moins un point d'aspiration (39) connecté à la source de vide (7) est présent dans ou sur ledit au moins un logement de canule (6), en particulier dans ou sur chaque logement de canule (6), respectivement, et dans lequel au moins un point d'aspiration (39) connecté à la source de vide (7) est de préférence présent dans ou sur chaque section de logement (24, 25) dudit au moins un logement de canule (6), respectivement.

15. Dispositif de fourniture (1) selon l'une quelconque des revendications précédentes, dans lequel le moyen de levage (17) peut être introduit à l'intérieur du magasin de canules (3) par l'intermédiaire d'une ouverture de prélèvement (5) du magasin de canules (3) dans le but de soulever une réserve de canules se trouvant à l'intérieur du magasin de canules (3).

16. Dispositif de fourniture (1) selon l'une quelconque des revendications précédentes, dans lequel le magasin de canules (3) comprend au moins un moyen de sécurisation (19), en particulier une goupille de sécurité, qui permet d'obturer une ouverture de prélèvement (5) du magasin de canules (3).

17. Dispositif de fourniture (1) selon l'une quelconque des revendications précédentes, dans lequel le dispositif de fourniture (1) comprend un dispositif (26) de retrait et/ou de mise en place dudit au moins un moyen de sécurisation (19).

18. Dispositif de fourniture (1) selon l'une quelconque des revendications précédentes, dans lequel le chariot de prélèvement (3) présente deux champs de prélèvement qui présentent chacun au moins un logement de canule (6) pour une canule (2), et est mobile sur une course pendulaire entre deux positions de transfert prévues sur des côtés différents du magasin de canules (3).

19. Procédé de fourniture de canules (2) pour une étape de procédé exécutée en aval lors du traitement de canules (2), en particulier lors de la fabrication de seringues, en utilisant un dispositif de fourniture (1) selon la revendication 2 ou selon l'une quelconque des revendications 3 à 18 en se référant à la revendication 2, dans lequel des canules (2) sont prélevées à partir du magasin de canules (3) du dispositif de fourniture (1) par l'intermédiaire d'un chariot de prélèvement (4), dans lequel la position d'au moins une canule (2) à l'intérieur dudit au moins un logement de canule (6) du chariot de prélèvement (4) est alors détectée à l'aide d'au moins un capteur (11), et dans lequel la canule (2) est ensuite amenée dans une position de consigne à l'aide du dispositif de correction de position (9) du dispositif de fourniture (1) en fonction d'un écart de position de la canule (2) par rapport à la position souhaitée détecté à l'aide du capteur (11) et d'une unité de commande (12) du dispositif de fourniture (1).

20. Procédé selon la revendication précédente, dans lequel ladite au moins une canule (2) est prélevée à l'aide d'une pince de prélèvement (15) du logement de canule (6) du chariot de prélèvement (4) et est transférée vers une étape de procédé exécutée en aval.

21. Programme informatique (34) contenant des instructions pour que le dispositif d'approvisionnement (1) selon la revendication 2 ou selon l'une quelconque des revendications 3 à 18 en se référant à la revendication 2, exécute le procédé selon l'une quelconque des revendications 19 ou 20, lorsque le programme est mis en oeuvre par un ordinateur qui commande le dispositif d'approvisionnement (1).

22. Support lisible par un ordinateur (33) sur lequel le programme d'ordinateur selon la revendication précédente est enregistré.
